Europäisches Patentamt

European Patent Office

Office européen des brevets

(19)

(11) Publication number: **0 080 882**
**B1**

(12) **EUROPEAN PATENT SPECIFICATION**

(45) Date of publication of patent specification: **04.02.87**

(21) Application number: **82306326.8**

(22) Date of filing: **26.11.82**

(51) Int. Cl.⁴: **B 65 D 47/28,** B 65 D 47/26,
B 65 F 1/16, A 61 M 5/00

(54) **Waste disposal bin.**

(30) Priority: **26.11.81 GB 8135722**

(43) Date of publication of application:
**08.06.83 Bulletin 83/23**

(45) Publication of the grant of the patent:
**04.02.87 Bulletin 87/06**

(84) Designated Contracting States:
**AT BE CH DE FR GB LI NL SE**

(56) References cited:
**US-A-1 720 551
US-A-1 963 050
US-A-2 202 653
US-A-3 749 274
US-A-3 792 803
US-A-4 275 628**

(73) Proprietor: **Owen, William Rees
56 Heol Llanishan Fach Rhiwbina
Cardiff, CF4 6LF (GB)**
(73) Proprietor: **Juhasz, Andras
53 Waterloo Road
Cardiff, CF3 7BJ (GB)**

(72) Inventor: **Owen, William Rees
56 Heol Llanishan Fach Rhiwbina
Cardiff, CF4 6LF (GB)**
Inventor: **Juhasz, Andras
53 Waterloo Road
Cardiff, CF3 7BJ (GB)**

(74) Representative: **Bernard, Alan Peter et al
F.J. CLEVELAND & COMPANY 40/43 Chancery
Lane
London WC2A 1JQ (GB)**

Courier Press, Leamington Spa, England.

## Description

This invention relates to containers adapted to be used for receiving waste products.

Bins and containers for receiving waste products are very well known, and a typical domestic waste bin is operated by a pedal which lifts the lid to enable waste to be deposited inside. The lid is usually arranged such that when the operator's foot is removed from the pedal the lid falls under its own weight to cover the opening to the bin. Another type has a pivoted roof portion which can swing about its pivot, in some cases on either side of the pivot, to reveal an entrance to the interior of the bin. The roof portion is pivoted and/or weighted so that it naturally falls back to an equilibrium position covering the entrance to the bin.

Waste disposal bins for hospitals however are required to have additional features which make the normal domestic bins not entirely suitable. One feature is that since the bins may contain combinated material, they should not have to be emptied by removing plastics lining bags, or by any other operation likely to involve spillage. It is preferable that the bin together with its contents should be incinerated when full. This means that the bin will have to be transported when full, and either of the types of domestic bins described above would be liabe to spill its contents if dropped or tilted. Plain cardboard drums have been used in hospitals to receive waste material, which is introduced through an aperture in the drum. When the drum is to be incinerated the aperture is plugged with a plastics bung. However use of this type of waste container means that while in use there is a direct opening to the contents of the container, and this is unacceptable when contaminated materials are likely to be amongst the contents. It is an object of the present invention to provide a waste disposal container which can be firmly closed for transportation and normally closed when in use but still able to be opened for use without difficulty.

United States Patent Specification No. 3,792,803 (Kessler) describes a self-reclosing cap for a dispensing container of small size. The cap covers an aperture into a receptacle of the container. The cap is guided for sliding motion between open and closed positions. A resiliently deformably member in the form of an elastic cord engages the receptacle and cap requiring the cord to be stretched to move the cap to reveal the aperture.

Such an arrangement is however not applicable to bins for waste disposal and other similar purposes which typically have a capacity of 5 litres. The degree of movement that is required of the closure member in such bins make the use of a stretchable elastic cord inapplicable. Furthermore, such bins are required to be disposed of along with their contents usually by incineration. They must therefore be cheap to manufacture. The object of the invention is to provide such a bin as cheaply as possible. Not only is the container described in U.S. 3,792,803 unsuitable for use on a larger scale, but it has the fundamental limitation that if the cap and resiliently deformable member are to be made as one-piece, the container must be a very small one. This is because the cap, made from the same elastic stretchable material as the deformable member, would be likely also to be stretched, to distort and generally be unsuitable as a sliding element. The document recognises the desirability of a one-piece construction but is fundamentally unable to provide it on the scale required for a waste disposal bin.

U.S. Patent Specification 3 749 274 describes a waste receiving receptacle with a hinged lid, the lid is biased by a leaf spring towards a closed position and the lid is depressable to reveal the opening to the receptacle. This arrangement however does not provide for a secure closure that will stand transportation when full without a risk of spillage. The leaf spring must always bear the weight of the lid and simple pressure down on the lid will open it. Furthermore, the document describes the mode of use of such a bin in an example in which a wet diaper is dropped on to the lid and this alone is sufficient to deflect the lid to allow the diaper to drop through. Such a procedure would be highly inappropriate if the waste product was itself a health hazard, or if the environment in which it was used, e.g. in a hospital, was such that exposed surfaces should be kept clean. Even if the lid was opened by hand prior to dropping the article, there is a clear likelihood that the users hand would be dirtied in the process.

In accordance with the present invention a closure member is constrained for rectilinear sliding movement across the plane of an aperture to a receptacle, between open and closed positions, a resilient deformable member, which is substantially unstretched during its operation, has a first preset natural shape and predisposition, when the closure member is in its closed position, and a second shape, when the closure member is in its open position, which is a distortion of the first shape, and from which the deformable member naturally returns to said first shape to close the container.

The container may have in working association therewith a device for rendering unusable a hypodermic syringe or similar article comprising a body member, and one or more blades supported for slicing movement beneath the plate member, the body member having at least one aperture dimensioned to receive the needle or barrel portion of the syringe such that by operating said one or more blades the needle or portion of the syringe is crushed or severed.

The device may be included as a removable insert in the aperture of the container.

Embodiments of the invention will now be described, by way of example only, with reference to the accompanying drawings.

In the drawings:—

Figure 1 is an axial sectional view through a drum forming the base of a waste disposal container;

Figure 2 is a plan view of the lid for the drum;

Figure 3 is an axial sectional view through the lid of Figure 2 along the line A—A;

Figure 4 is an axial sectional view through the lid of Figure 2 along the line B—B;

Figure 5 is an enlarged part of the sectional view of Figure 4;

Figure 6 is a plan view of a closure cap for the lid;

Figure 7 is an end elevation view of the closure cap;

Figure 8 is a side elevation of the closure cap;

Figure 9 is a scrap view in the direction arrow A;

Figure 10 is an axial section through a device for crushing syringes;

Figure 11 is a plan view of the device of Figure 10;

Figure 12 is a plan view of one blade incorporated in the crusher device.

Referring to the Figures, a waste disposal container for medical waste products if formed from three interfitting parts. These parts are a drum 1 which forms the base of the container, a lid 2 and a closure cap 3.

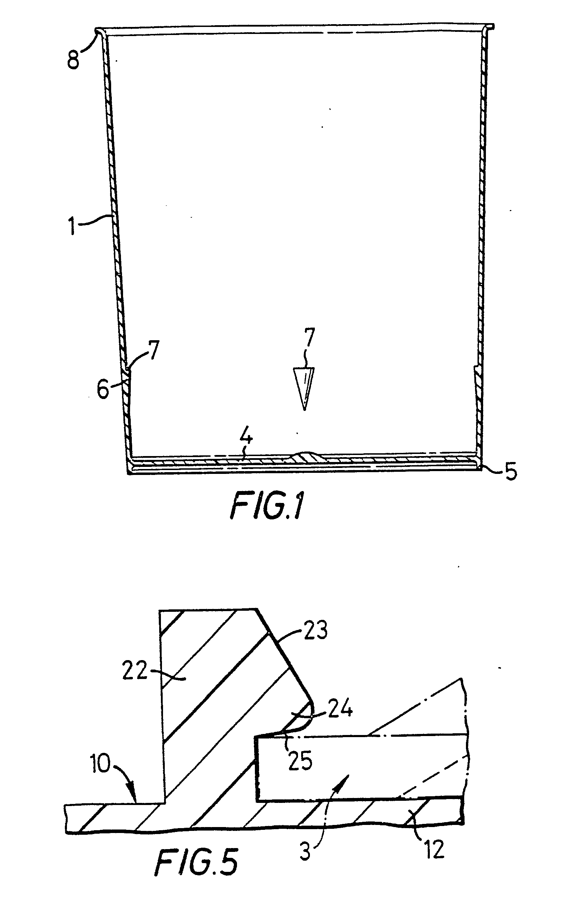

Referring in particular to Figure 1, the drum 1 is a thin walled, hollow structure of circular cross-section, and is slightly tapered from top to bottom. The narrower end of the drum is closed by an integrally formed base wall 4 whose inward-facing surface forms the bottom surface of the container. The side wall of the drum 1 extends by a small amount beneath the base wall 4 to form an annular flange 5 on which the container stands when in use. The inner surface of the side wall of the drum 1 has integrally formed therewith at a short distance up from the base wall 4, four-equally spaced lugs 6 of wedge-shape cross-section, disposed such that the each presents a narrow circumferential ledge 7 facing the open upper end of the drum. This enables another drum to be stacked with its base resting on the ledge 7.

At the open, wider end of the drum the side wall is bent outwardly to give a narrow, circular lip 8 the function of which is to engage the lid 2, as is described below.

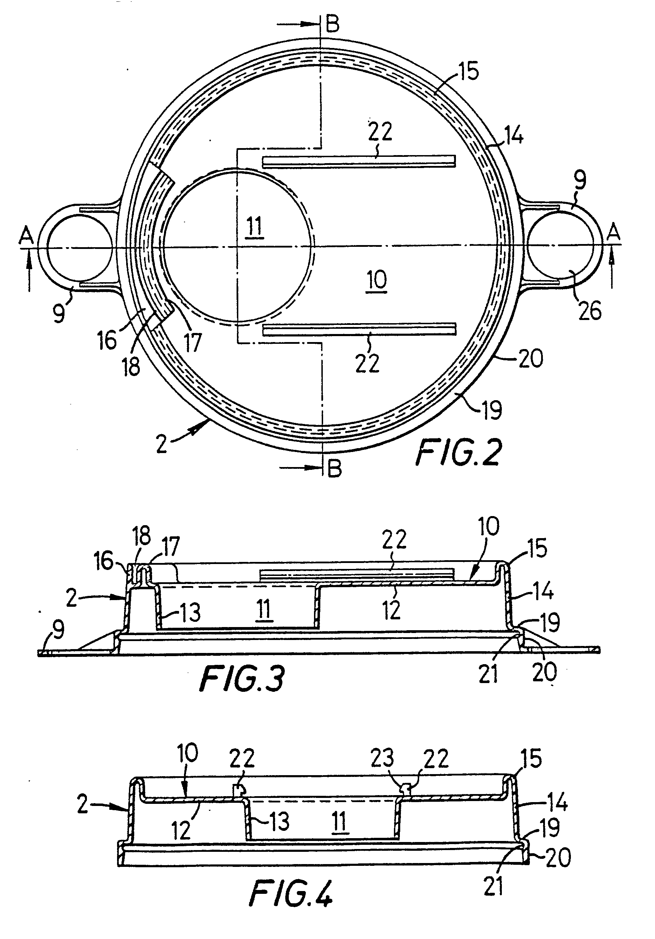

Referring now to Figures 2, 3 and 4, the lid 2 is generally circular in plan with two lugs 9, described in more detail below. The transverse surface 10 of the lid is generally circular in plan and in one half of its surface it includes a circular aperture 11 through which waste products are introduced into the assembled container. The aperture 11 is located between a position close to a diameter of the transverse surface 10 and a position a small distance from the edge of the surface 10. The transverse surface 10 is the upper surface of a wall made of polypropylene or other suitable plastics material 12 which is integral with an annular skirt 14, perpendicular to the surface 10 and extending downwards along the periphery of the circular aperture 11. The side wall 14 of the lid 2 is integrally moulded with the upper wall 12. The upper wall 12 and the side wall 14 are joined by an upstanding flange 15 comprising a portion extending upwardly from the periphery of the upper wall 12 which is smoothly curved at its upper end and folded back on itself, and is then continuous with the circumferential side wall of the lid 2. Thus the upstanding flange 15 has a double thickness of material separated by a small channel. The upstanding flange 15 extends around the greater part of the circumference of the transverse surface 10 of the lid, apart from a region of the circumference adjacent the radially outer edge of the circular aperture 11. In this region of the periphery of the transverse surface of the lid the two walls of the upstanding flange 15 reduce to a single, thicker wall 16. The thickness of the wall 16 varies along its length so that its radially inner surface is coaxial with the aperture 11.

Between the wall 16 and the edge of the circular aperture 11 the upper wall 12 of the lid is subject to an upward fold so that an arcuate double-walled ridge 17 is located between the wall 16 and the aperture 11. The ridge 17 has the same height as the wall 16 and is disposed along a circular arc co-axial with the circular aperture 11. The ridge 17 extends along an arc subtending approximately 80° and defines an arcuate channel 18 between itself and the wall 16. The channel 18 is thus also disposed along an arc co-axial with the circular aperture 11 and subtends an angle of 80°. The upper parts of the wall 16 and ridge 17 facing inwards towards the slot 18 are slightly thickened so that the slot is of reduced width adjacent its open end.

The circumferential side wall 14 of the lid extends downwardly from the upstanding flange 15, or from the single-thickness wall 16 in the region adjacent the circular aperture 11 and is tapered so that the diameter of the lid increases towards at its lower edge. Nearer the lower end of the circumferential side wall 14 the wall projects outwardly to form a step 19 so that the diameter of the lid within the portion 20 of the side wall 14 below the step is greater than the diameter of the lid in the region above the step. The lower wall portion 20 reduces in thickness from below the step to its lower edge, but just adjacent the step 19 has a small circumferential recess 21 which is adapted to receive the rim of the drum portion of the container.

Two elongate rails 22 are located on the transverse surface 10 of the upper wall 12 of the lid. These rails 22 are parallel and their inner facing edges are generally tangential to the edge of the circular aperture 11. The rails 22 extend from just short of the periphery of the lid on the side opposite the single-thickness wall 16 and channel 18 and terminate just short of that diameter of the circular aperture 11 which is perpendicular to the length of the rails. The sides of the rails 11 upstanding from the transverse surface 10 and facing each other across the surface are contoured to provide engagement strips 23 which are adapted to engage the closure cap of the container as is further described below.

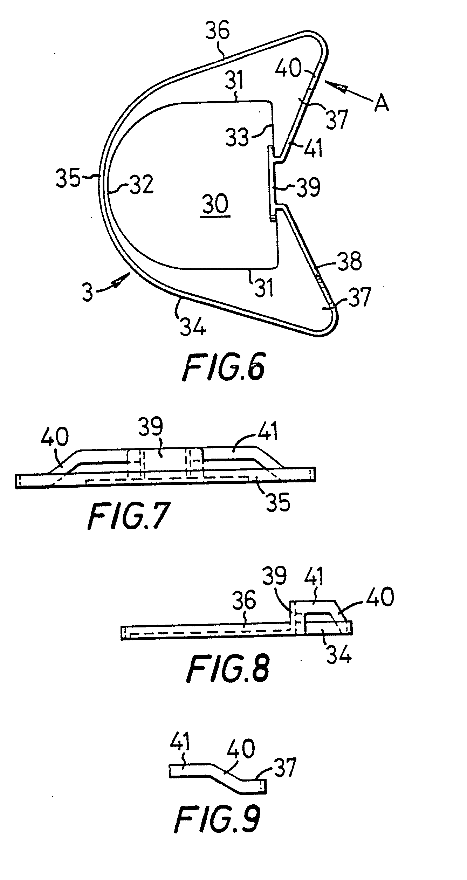

Figure 5 shows, in enlarged scale, one of the rails 22 and illustrates the engagement strip 23 which is formed by an angled protuberance 24 from the top edge of the rail to provide the inwards-facing wall of the rail with a 30° slope. This angled surface terminates approximately one third of the way down the inwards-facing surface of the rail and is smoothly rounded and continuous with the under surface 25 of the engagement strip. The under surface 25 is at an angle of 100° to the upstanding side surface of the rail and is therefore slightly upwardly inclined. The under surface 25 forms an abutment surface to maintain the closure cap 3 in position.

The lugs 9 extend from the outer periphery of the lid at diametrically opposite positions. One of the lugs is symmetrically disposed adjacent the single-thickness wall 16 so that the lugs 9 are also diametrically disposed with respect to the circular aperture 11. Each lug is a flat transverse projection from the lower edge of the circumferential side wall 16 and each lug encloses a circular aperture 26 which occupies the major part of its area. The lugs 9 are used for lifting the container and also prevent the container from rolling if knocked over.

Referring now to Figures 6, 7, 8 and 9, the closure cap 3 comprises a cover plate 30 having a pair of parallel sides 31 and a semi-circular front end 32 and a rectilinear rear edge 33. The cover plate 30 has a configuration adapting it to cover the circular aperture 11 in the lid 2. The cover plate is fixed within a skeletal outer frame 34. The frame 34 is a narrow strip of low density polypropylene disposed edge-on with respect to the plane of the cover plate 30. The outer frame 34 has an arcuate frontal portion 35 with a radius somewhat greater than the semi-circular front end 32 of the cover plate 30 and is situated forward of the front end of the cover plate and spaced therefrom by a narrow gap. The sides 36 of the outer frame diverge towards the rear of the cover plate and extend rearwardly beyond the rear edge of the cover plate. At their rear extremities the sides of the frame turn inwards and forwards to form two swept-back wings 37. The in-turned parts 38 of the wings are connected above the rear edge of the cover plate by a rectangular plate 39 upstanding from the rear edge 33 of the cover plate.

As can be best seen from Figures 7 and 8, the wings 37 are not coplanar with the front portion of the skeletal frame 34; the in-turned parts 38 include an upwards sloping portion 40 at a position spaced inwardly of the arcuate tips of the wings. The edges of the inner portion of each in-turned part 38 of the wings are horizontal and the inner portions 41 are bent at their ends to join the plate 39 perpendicularly.

The container is assembled by pressing the lid 2 over the open end of the drum 1. The lip 6 around the upper edge of the drum gives the drum a slightly wider diameter than the internal radius of the lower wall portion 20 of the lid so that as the lid is pushed down it is resiliently deformed as the lip 6 is pressed between the thicker parts of the lower wall portion 20 until it engages in the circumferential recess 21, where it engages as a snap fit.

The closure cap 3 is fitted onto the lid 2 by pressing the cover plate 30 between the engagement strips 23. This action resiliently deforms the rails 22. The arcuate frontal portion 35 of the outer frame of the closure cap is positioned in the slot 18.

To reveal the aperture 11 the cover plate is pulled away from the ridge 17, and the arcuate frontal portion 35 of the frame remains in the slot 18. The consequent resilient deformation of the skeletal frame 34 produces a returning force tending to return the cover plate to a position in which it completely covers the aperture 11. The cover plate 30 can move forwards and backwards within the rails 22 between a position where it covers the aperture 11 to a position where the whole of the aperture is opened.

The pulling movement necessary to open the aperture 11 is accomplished by pressing against the plate 39 thus deforming the wings 37 of the skeletal frame. The cap, and in particular the frame portion, is of a size such that a user can conveniently pull the frame rearwardly by means of his thumb and forefinger so that opening and closing can be accomplished with the use of one hand only. An item for disposal can therefore be held in the free hand whilst the aperture is opened. It will be appreciated that this is particularly advantageous since it enables the user to complete the waste disposal operation without having to rest the, possible infected, waste article on any other surface.

The container can thus be repeatedly opened and closed during use so that when only partially full does not normally present an opening from the interior to the local environment. The same resilient closure mechanism is also used when the bin is subsequently carried away for incineration and therefore there is a significant economy in the mechanisms that need to be employed.

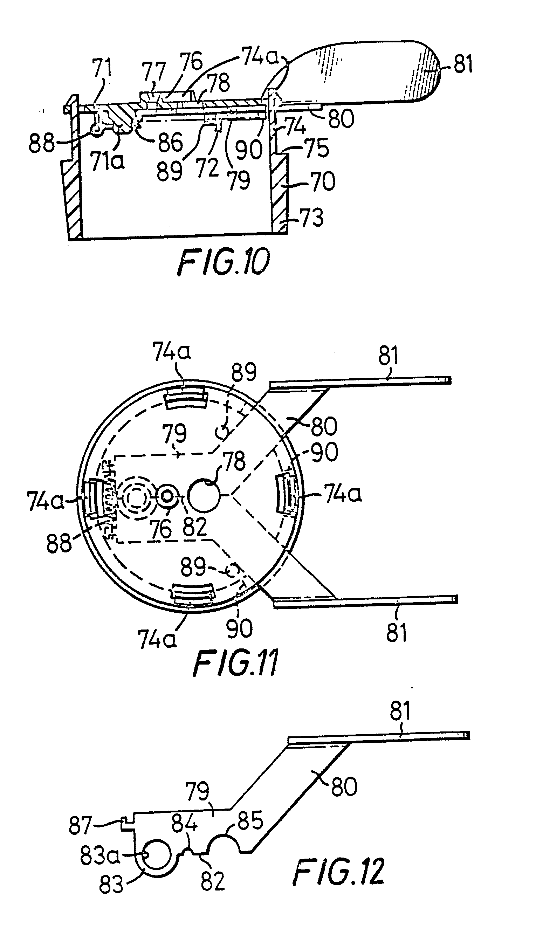

Referring to Figures 10 to 12 a crusher device for use with the waste disposal bin described with reference to Figures 1 to 9 comprises a barrel 70, a cover plate 71, and a pair of stainless steel slicing arms 72. The device is adapted to fit as an insert in the circular aperture 11 in the lid of the bin. The barrel 70, moulded from co-planar polypropylene, is generally cylindrical in shape. The wall of the barrel comprises lower and upper cylindrical portions 73 and 74, the former being thinner than the latter. The inner surfaces of the two portions are flush so that an annular step 75 results on the outer surface. The thickness of the lower portion 73 tapers slightly in a downwards direction and the external diameter of the barrel at its lower extremity is approximately equal to that of the lower periphery of the skirt 13 around the aperture 11. The barrel may thus be push-fitted into the aperture 11 so that the step 75 is flush with the transverse surface 10 of the lid of the bin.

The cover plate 71 is circular in outline and has four equiangularly-spaced arcuate slots near its periphery and centred about the centre of the plate. Four corresponding lugs 74a at the upper periphery of the upper portion 74 of the barrel enable the cover plate to be securely fixed to the barrel. An integral boss 76 upstands from the surface of the cover plate and has a bore 77 arcuately tapering from an upper entrance opening to a lower narrow outlet. The cover plate has a further entrance aperture 78 to one side of the boss 76. On the other side of the boss, in line with the boss and the aperture 78 is a downwardly extending peg 71a, about which the slicing arms 72 are rotatably mounted.

The two slicing arms 72 each comprise a horizontal, flat stem portion 79, and outwardly-angled portion 80, and a lateral, operating plate 81 upstanding from the end of the angled portion 80. The longitudinal axis of the operating plate 81 being parallel to, but spaced outwardly from the stem portion 79. The longitudinal edge 82 of the stem portion that is remote from the operating plate 81 has a projecting ear 83 adjacent its free end. The ear 83 partially surrounds a circular aperture 84 through the stem portion. Spaced from the ear 83 the edge 82 has a small semi-circular recess 84 and further spaced therefrom another, larger, semi-circular recess 85.

The two arms 72 are oppositely handed and are aligned beneath the cap such that their edges 82 are aligned, but with one arm being slightly above the other so that by moving the arms horizontally a slicing action takes place.

The arms are mounted by inserting the peg 71a through the overlapping apertures 83a in the arms. A washer and spire fixing 86 retains the arms on the peg enabling them to pivot thereon.

At the free end of each stem portion 79 the arms include a bent tab portion 87. A tension spring 88 is interposed between the tab portions 87 forcing the angled portions outwardly against respective stops 89 extending downwards from the undersurface of the cap. The extent of the arms is accommodated by a pair of circumferential slots 90 through the upper wall portion of the barrel extending in an arc subtending the same angle as the stops 89.

The device is used to crush used syringes. It is located in the aperture 11 in the lid of the bin by fully retracting the cover plate 30. When released, the cover plate abuts against the outer surface of the upper wall of the barrel. In this position the cover plate 30 overlaps the step 75 preventing the barrel from being removed, unless by withdrawal of the cover plate. The device is used by inserting a used syringe through the boss 76 so that the needle projects through the hole formed by the two semi-circular recesses 84. The operating plates 81 of the arms are then squeezed together shearing the needle. When released, the arms are folded back against the stops by the action of the spring 88. The syringe is then withdrawn and reinserted through the entrance aperture 78 in the cap and then through the underlying hole formed by the semi-circular recesses 85 in the arms. By squeezing the arms the nozzle of the syringe is severed.

It will be appreciated that by means of these two operations the syringe is rendered completely useless. Once the waste bin is full the crusher device can be removed, cleaned and sterilized and repeatedly reused.

**Claims**

1. A self-closing container adapted to be used for receiving waste or contaminative articles comprising a receptacle (1) having an aperture (11) through which articles may pass to enable the articles to be deposited in the container, a closure member (3) secured to the receptacle a constrained for movement between first and second positions at which the aperture is opened and closed respectively, and means for releasably securing the closure member in its second position comprising a resiliently deformable member (34) engaged with the receptacle and with the closure member such that it has to be deformed against its resilience to move the closure member from its closed position, characterised in that said constraint of the closure member is for rectilinear sliding movement across the plane of the aperture and the resiliently deformable member is substantially unstretched during its operation and has a first, preset natural shape and predisposition, when the closure member is in its closed positions and a second shape, when the closure member is in its open position, which is a distortion of the first shape, and from which the deformable member naturally returns to said first shape to close the container.

2. A self-closing container as claimed in claim 1 characterised in that the closure member and the resiliently deformable member are formed as a single element.

3. A self-closing container as claimed in claim 2 characterised in that the single element comprising the closure member and resiliently deformable member is composed of a single material.

4. A container as claimed in any one of the preceding claims characterised in that the resiliently deformable member comprises an elongate member joined at both ends to the closure member and being secured to the container by being located by retaining means (17, 18) the elongate member comprising a band extending around the periphery of the closure member and joined to the closure member at the rear thereof, the band being located by said retaining means at a position closer to the end of the closure member which precedes in its movement to the closed position.

5. A container as claimed in any one of the preceding claims characterised in that it includes an operating surface (39) joined to the closure member and upstanding therefrom, the operating surface being adapted for use by an operator to apply pressure by hand to move the closure member from its second position.

6. A container as claimed in claim 4 characterised in that said retaining means comprises a slot (18) having at least one resiliently deformable sidewall, the slot being enabled to receive said band by the resilient deformation of said side wall or walls.

7. A container as claimed in any one of the preceding claims characterised by the inclusion, in working association therewith, of a device for rendering unusable a hypodermic syringe or similar article comprising a body member (70), and one or more blades (72) supported for slicing movement beneath the body member, the body member having at least one aperture dimensioned to receive the needle or barrel portion of a syringe such that by operating said one or more blades the needle or barrel portion of the syringe is crushed or severed.

8. A container as claimed in claim 7 characterised in that in said device the plate includes two apertures (77, 78), one dimensioned to receive a needle of a syringe and the other dimensioned to receive the barrel of a syringe such that by the partial insertion of the syringe through an appropriate one of the apertures the needle or the barrel respectively may be severed or crushed by operation of the blade or blades.

9. A container as claimed in claim 7 or claim 8 characterised in that said device includes a pair of said blades, the blades being pivotally mounted beneath said body member for movement in respective closely spaced parallel planes such that motion thereof constitutes a slicing action.

10. A container as claimed in claim 9 characterised in that in said device the slicing edges of the two blades are located beneath said apertures and one or both of said edges has or have recesses in (84, 85) in register with said two apertures for receiving the inserted needle or barrel respectively.

11. A container as claimed in claim 9 or claim 10 characterised in that in said device the pair of blades are biased towards the position in which their slicing edges are non-overlapping.

12. A container as claimed in any one of claims 7 to 11 characterised in that said one or more apertures of the device constitutes opening(s) into said receptacle.

13. A container as claimed in claim 12 characterised in that said device is a removable insert in said aperture of the container.

14. A container as claimed in claim 13, when characterised in that said device includes on its external surface a retaining ledge (75) extending on the container side of the plane of movement of the closure member, the closure member being biased by said resiliently deformable member into abutment with the inserted device and overlying said retaining ledge to prevent removal of the device.

**Patentansprüche**

1. Ein selbstschließender Behälter, benutzbar zur Aufnahame von Abfall oder anderer Gegenstände, enthaltend einen Behälter (1), der eine Öffnung (11) aufweist durch welche Gegestände hindurchgelangen können um diesen zu ermöglichen sich in dem Behälter abzulagern, ein am Behälter befestigtes Verschlußglied (3), ein zwangsgeführt zwischen ersten und zweiten Stellungen, bei denen die Öffnung jeweils geöffnet und geschlossen ist, bewegbare und Mittel zur lösbaren Befestigung des Verschlußgliedes in seiner zweiten Stellung, enthaltend ein elastisch verformbares Glied (34) welches mit dem Behälter und demn Verschlußglied verbunden ist, so daß es gegen seine Elastizität verformt werden muß um das Verschlußglied von seiner geschlossenen Stellung zu bewegen, dadurch gekennzeichnet, daß die besagte Zwangsführung des Verschlußgliedes einer geradlinigen Gleitbewegung über der Fläche der Öffnung entspricht und das elastisch verformbare Glied während seines Betriebes im wesentlichen entspannt ist und eine erste vorgegebene natürliche Form und Ausbildung hat, wenn das Verschlußglied in seinen geschlossenen Stellungen ist und eine zweite Form, wenn das Verschlußglied in seiner offenen Stellung ist, welche eine Verformung der ersten Form darstellt, und von der das verformbare Glied natürlich in die besagte erste Form zurückkehrt, um den Behälter zu schließen.

2. Ein selbstschließender Behälter nach Anspruch 1, dadurch gekennzeichnet, daß das Verschlußglied und das elastisch verformbare Glied als ein Teil ausgebildet sind.

3. Ein selbstschließender Behälter nach Anspruch 2, dadurch gekennzeichnet, daß das eine Teil, weches das Verschlußglied und das elastisch verformbare Glied enthält, sich aus einem einzigen Material zusammensetzt.

4. Ein Behälter nach einem der vorstehenden Ansprüche, dadurch gekennzeichnet, daß das besagte verformbare Glied ein längliches Glied enthält, welches an beiden Enden mit dem Verschlußglied verbunden und an dem Behälter befestigt ist, indem es durch Haltemittel (17, 18) fixiert wird, wobei das längliche Glied ein Band enthält, das um die Außenfläche des Verschlußgliedes verläuft und an der Rückseite davon mit dem Verschlußglied verbunden ist, wobei das Band durch die besagten Haltemittel in einer Stellung fixiert wird, die dem Ende des Verschlußgliedes näher ist, und in seiner Bewegung zur geschlossenen Stellung vorausgeht.

5. Ein Behälter nach einem der vorstehenden Ansprüche, dadurch gekennzeichnet, daß er eine Betätigungsfläche (39) enthält, die mit dem Verschlußglied verbunden ist und davon nach oben vorstehend, durch einen Benutzer nutzbar ist, um Druck mit der Hand auszuüben und so das Verschlußglied aus seiner zweiten Stellung zu bewegen.

6. Ein Behälter nach Anspruch 4, dadurch gekennzeichnet, daß die besagten Haltemittel einen Schlitz (18) enthalten, der mindestens eine elastisch verformbare Seitenwand aufweist, wobei der Schlitz imstande ist das besagte Band durch die elastische Verformung der besagten Seitenwand oder Wände aufzunehmen.

7. Ein Behälter nach einem der vorstehenden Ansprüche, gekennzeichnet durch die Einbeziehung einer damit in Arbeitsverbindung stehenden Einrichtung um eine hypodermatische Spritze oder einen ähnlichen Gegenstand unbrauchbar zu machen, enthaltend einen Grundkörper (70) und einen oder mehrere zur schneidenden Bewegung unterhalb des Grundkörpers gehaltene Schneiden, wobei der Grundkörper mindestens eine Öffnung aufweist die so bemessen ist, das sie die Nadel oder das Zylinderteil eine Spritze aufnimmt, so daß durch den Betrieb die besagte eine oder mehrere Schneiden die Nadel oder das Zylinderteil der Spritze zermalmt oder abgetrennt wird.

8. Ein Behälter nach Anspruch 7, dadurch gekennzeichnet, daß in der besagten Vorrichtung die Platte zwei Öffnungen (77, 78) enthält, von denen eine so bemessen ist, daß sie eine Nadel einer Spritze aufnimmt und die andere so bemessen ist, daß sie den Zylinder einer Spritze aufnimmt, so daß durch die teilweise Einführung der Spritze über eine geeignete Vorrichtungen die Nadel bzw. der Zylinder durch die Wirkung der Schneide oder Schneiden losgetrennt oder zermalmt werden können.

9. Ein Behälter nach Anspruch 7 oder 8, dadurch gekennzeichnet, daß die besagte Vorrichtung ein Paar der besagten Schneiden enthält, wobei die Schneiden unterhalb des besagten Grundkörpers drehbar gelagert sind zur Bewegung in jeweils dicht nebeneinander angeordneten parallelen Ebenen, so daß ihre Bewegung eine Schneidwirkung erzeugt.

10. Ein Behälter nach Anspruch 9, dadurch gekennzeichnet, daß in der besagten Vorrichtung die schneidenden Kanten der beiden Schneiden sich unterhalb der besagten Öffnungen befinden und eine oder beide der besageten Kannten Vertiefungen in (84, 85) hat oder haben fluchtend mit besagten zwei Öffnungen zur jeweiligen Aufnahme der eingeworfenen Nadel oder des Zylinderes.

11. Ein Behälter nach Anspruch 9 oder Anspruch 10, dadurch gekennzeichnet, daß in der besagten Vorrichtung das Paar der Schneiden gegen die Stellung vorgespannt ist, in der ihre schneidenden Kanten sich nicht überlappen.

12. Ein Behälter nach einem der Ansprüche 7 bis 11, dadurch gekennzeichnet, daß das besagte eine oder mehrere Geräte der Vorrichtung Öffnung(en) in den besagten Behälter hinein bildet.

13. Ein Behälter nach Anspruch 12, dadurch gekennzeichnet, daß die besagte Vorrichtung ein auswechselbarer Zusatz in dem besagten Gerät des Behälters ist.

14. Ein Behälter nach Anspruch 13 enthält, wenn er dadurch gekennzeichnet ist, daß diese besagte Vorrichtung auf seiner Außenfläche eine Halteleiste (75) enthält, die auf der Behälterseite oder Bewegungsebene des Verschlußgliedes verläuft, wobei das Verschlußglied durch das besagte elastisch verformbare Glied zur Anlage an der eingeführten Vorrichtung vorgespannt ist, und die besagte Halteleiste überlappt um das Herausnehmen der Vorrichtung zu verhindern.

**Revendications**

1. Récipient à auto-fermeture adapté pour être utilisé pour recevoir des déchets ou des articles contaminateurs comprenant un réceptacle (1) ayant une ouverture (11) à travers laquelle les articles peuvent passer pour permettre aux articles d'être déposés dans le récipient, un élément de fermeture (3) fixé au réceptacle et contraint pour un déplacement entre des première et seconde positions auxquelles l'ouverture est ouverte et fermée respectivement, et un moyen pour fixer de façon relâchable l'élément de fermeture dans sa seconde position comprenant un élément élastiquement déformable (34) engagé avec le réceptacle et avec l'élément de fermeture de sorte qu'il doit être déformé contre son élasticité pour déplacer l'élément de fermeture de sa position fermée, caractérisé en ce que ladite contrainte de l'élément de fermeture est pour un déplacement coulissant rectiligne à travers le plan de l'ouverture et l'élément élastiquement déformable est sensiblement non tendu pendant son fonctionnement et a une première forme naturelle préétablie et une prédisposition, lorsque l'élément de fermeture est dans sa position fermée et une seconde forme, lorsque l'élément de fermeture est dans sa position ouverte, qui est une déformation de la première forme, et à partir de laquelle l'élément déformable retourne naturellement à ladite première forme pour fermer le récipient.

2. Récipient à auto-fermeture selon la revendication 1, caractérisé en ce que l'élément de fermeture et l'élément élastiquement déformable sont formés en un seul élément.

3. Récipient à auto-fermeture selon la revendication 2, caractérisé en ce que le seul élément comprenant l'élément de fermeture et l'élément élastiquement déformable est composé d'un seul matériau.

4. Récipient selon l'une des revendications précédentes caractérisé en ce que l'élément élastiquement déformable comprend un élément allongé joint aux deux extrémités à l'élément de fermeture et étant fixé au récipient en étant situé par des moyens de retenue (17, 18) l'élément allongé comprend une bande s'étendant autour de la périphérie de l'élément de fermeture et jointe à l'élément de fermeture à l'arrière de celui-ci, la bande étant située par ledit moyen de retenue à une position proche de l'extrémité de l'élément de fermeture qui précède dans son mouvement la position fermée.

5. Récipient selon l'une des revendications précédentes caractérisé en ce qu'il comprend une surface d'actionnement (39) jointe à l'élément de fermeture et s'élevant de celui-ci, la surface d'actionnement étant adaptée pour utilisation par un opérateur pour appliquer une pression à la main pour déplacer l'élément de fermeture de sa seconde position.

6. Récipient selon la revendication 4, caractérisé en ce que le moyen de retenue précité comprend une fente (18) ayant au moins une paroi latérale élastiquement déformable, la fente permettant de recevoir la bande précitée par la déformation élastique de la paroi latérale ou des parois.

7. Récipient selon l'une des revendications précédentes, caractérisé par l'inclusion, en association de fonctionnement avec celui-ci, d'un dispositif pour rendre inutilisable une seringue hypodermique ou article similaire comprenant un élément formant corps (70) et une ou plusieurs lames (72) supportées pour un mouvement tranchant sous l'élément formant corps, l'élément formant corps ayant au moins une ouverture dimensionnée pour recevoir l'aiguille ou la partie cylindrique d'un seringue telle que par l'actionnement de l'une ou plus des lames l'aiguille ou la partie cylindrique de la seringue soit écrasée ou cassée.

8. Récipient selon la revendication 7 caractérisé en ce que dans le dispositif précité la plaque comprend deux ouvertures (77, 78), une dimensionnée pour recevoir une aiguille d'une seringue et l'autre dimensionnée pour recevoir le cylindre d'une seringue de sorte que par l'insertion partielle de la seringue à travers une appropriée des ouvertures l'aiguille ou le cylindre peut être respectivement cassé ou écrasé par l'actionnement de la lame ou des lames.

9. Récipient selon la revendication 7 ou la revendication 8, caractérisé en ce que le dispositif précité comprend deux lames, les lames étant montés pivotantes sous l'élément formant corps précité pour un mouvement dans des plans respectifs parallèles espacés de façon proche de sorte que le déplacement de celui-ci constitue une action tranchante.

10. Récipient selon la revendication 9, caractérisé en ce que dans le dispositif précité les bords tranchants des deux lames sont situés sous les ouvertures et l'un ou les deux des bords a ou ont des évidements (84, 85) en coïncidence avec les deux ouvertures pour recevoir l'aiguille insérée ou le cylindre respectivement.

11. Récipient selon la revendication 9 ou la revendication 10, caractérisé en ce que dans le dispositif précité les deux lames sont sollicitées vers la position dans laquelle leurs bords tranchants ne se chevauchent pas.

12. Récipient selon l'une des revendications 7 à 11, caractérisé en ce que une ou plus des ouvertures du dispositif constitue une (des) ouverture(s) dans le réceptacle précité.

13. Récipient selon la revendication 12, caractérisé en ce que le dispositif précité est une pièce rapportée amovible dans l'ouverture précitée du récipient.

14. Récipient selon la revendication 13, caractérisé en ce que le dispositif précité comprend sur sa surface externe un rebord de retenue (75) s'étendant sur le côté du récipient du plan de déplacement de l'élément de fermeture, l'élément de fermeture étant sollicité par l'élément élastiquement déformable précité en aboutement avec le dispositif inséré et au-dessus dudit rebord de retenue pour empêcher le retrait du dispositif.

0 080 882

FIG.1

FIG.5

1

FIG.2

FIG.3

FIG.4

FIG.6

FIG.7

FIG.8

FIG.9

FIG.10

FIG.11

FIG.12

4